(19) Europäisches Patentamt
European Patent Office
Office européen des brevets

(11) Publication number: **0 090 340 A1**

# (12) EUROPEAN PATENT APPLICATION

(21) Application number: 83102866.7

(22) Date of filing: 23.03.83

(51) Int. Cl.³: **C 07 D 205/08**, C 07 F 7/10

(30) Priority: 27.03.82 JP 49527/82

(43) Date of publication of application: 05.10.83 Bulletin 83/40

(84) Designated Contracting States: BE CH DE FR GB IT LI NL

(71) Applicant: KANEGAFUCHI KAGAKU KOGYO KABUSHIKI KAISHA, 2-4 Nakanoshima 3-chome, Kita-ku Osaka-shi (JP)

(72) Inventor: Umezawa, Hamao, 23, Toyotama Kita 4-chome, Nerima-ku Tokyo (JP)
Inventor: Shimazaki, Masami, 8-4, Nishihata 3-chome, Takasago-shi Hyogo-ken (JP)
Inventor: Ideguchi, Satoshi, 7-20, Miyamadai 3-chome Tarumi-ku, Kobe-shi Hyogo-ken (JP)
Inventor: Murakami, Hiroshi, 2-63, Okihama-cho Takasago-cho, Takasago-shi Hyogo-ken (JP)
Inventor: Nagashima, Nobuo, 2-63, Okihama-cho Takasaga-cho, Takasago-shi Hyogo-ken (JP)
Inventor: Ueyama, Noboru, 2-63, Okihama-cho Takasago-cho, Takasago-shi Hyogo-ken (JP)
Inventor: Ohashi, Takehisa, 1-9-403, Tsurukabuto 4-chome Nada-ku, Kobe-shi Hyogo-ken (JP)
Inventor: Watanabe, Kiyoshi, 15-41, Matsugaoka 5-chome, Akashi-shi Hyogo-ken (JP)
Inventor: Ohno, Masaji, 1565-13, Koshigoe Kamakura-shi, Kanagawa-ken (JP)

(74) Representative: Türk, Dietmar, Dr. rer. nat. et al, Redies, Redies, Türk & Gille Patentanwälte Brucknerstrasse 20, D-4000 Düsseldorf 13 (DE)

## (54) Process for preparation of optically active 4-(2-Hydroxyethyl)-2-azetidinone.

(57) An improved process for preparing an optically active 4-(2-hydroxyethyl)-2-azetidinone represented by the formula (I):

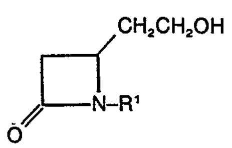

(I)

wherein $R^1$ is hydrogen atom or a protective group of amino group, by reacting an optically active carboxylic acid ester represented by the formula (II):

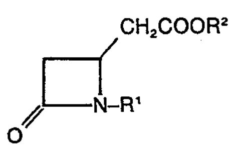

(II)

wherein $R^1$ is the same as that defined above; $R^2$ is alkyl or aralkyl group having from 1 to 7 carbon atoms, with a reducing agent. The product is an important and useful intermediate compound for producing a known or novel class of antibiotics such as thienamycin or its analogs.

EP 0 090 340 A1

PROCESS FOR PREPARATION OF OPTICALLY ACTIVE 4-(2-HYDROXYETHYL)-2-AZETIDINONE

The present invention relates to a process for the preparation of an optically active 4-(2-hydroxyethyl)-2-azetidinone. More specifically, the present invention relates to a process for preparing an optically active 4-(2-hydroxyethyl)-2-azetidinone represented by the formula (I):

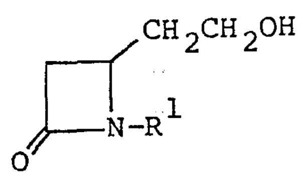

(I)

wherein $R^1$ is hydrogen atom or a protective group of amino group,
which comprises reacting an optically active carboxylic acid ester represented by the formula (II):

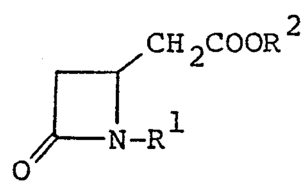

(II)

wherein $R^1$ is the same as that defined above; $R^2$ is alkyl or aralkyl group having from 1 to 7 carbon atoms, with a reducing agent.

The product of the present invention is an important and useful intermediate compound for producing a known or novel class of antibiotics. For example, thienamycin and its analogs are produced from the compound of the present invention.

An optically active starting compound (II) has been available by chemico-enzymatic reaction [M. Ohno, et al, J. Am. Chem. Soc., 103, 2405 (1981)] and the establishment of the method to convert the side chain at C-4 position of the compound (II) into 4-(2-hydroxyethyl) group while maintaining its configuration has been

expected to develope an easy method for producing thienamycin or its analogs.

The object of the present invention is therefore to provide an improved process for preparing an optically active 4-(2-hydroxyethyl)-2-azetidinone by reacting an optically active carboxylic acid ester with a reducing agent. A racemic compound of the formula (I) is obtained from 4-(2-acetyloxyethyl)-2-azetidinone by alkaline hydrolysis [e.g. U.S.P. Nos. 4,217,453; 4,218,459; 4,18,463], but the preparation of an optically active compound (I) has never hitherto been disclosed. A racemic 4-hydroxymethyl-2-azetidinone being a homologous compound of the compound (I) is prepared by reduction of 4-methoxycarbonyl-2-azetidinone with sodium boron tetrahydride [W.F. Huffman, K.G. Holden, T.F. Buckley, J.G. Gleason, L. Wu, J. Am. Chem. Soc., 99, 2352 (1977)]. The reduction of the compound (II) with sodium boron tetrahydride, however, gives only a little amount of the desired compound (I).

The present inventors found that a more reactive reducing agent than sodium boron tetrahydride, e.g., lithium boron tetrahydride is effective for converting an ester group in the compound (II) into an alcohol group. Such reagents as more reactive than sodium boron tetrahydride were thought to be so reactive that an unstable β-lactam ring might be cleaved. In fact, there was no previous example of using such reagents in this field.

In accordance with the present invention, there can be provided a process for the preparation of an optically active 4-(2-hydroxyethyl)-2-azetidinone represented by the formula (I):

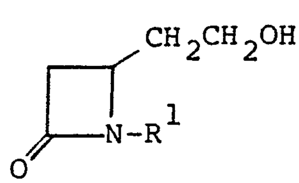

(I)

wherein $R^1$ is hydrogen atom or a protective group of amino group,

which comprises reacting an optically active carboxylic acid ester represented by the formula (II):

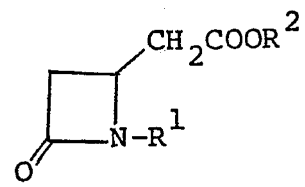

(II)

wherein $R^1$ is the same as that defined above; $R^2$ is alkyl or aralkyl group having from 1 to 7 carbon atoms, with a reducing agent.

As previously noted, the present invention involves an improved process for the preparation of an optically active 4-(2-hydroxyethyl)-2-azetidinone represented by the formula (I):

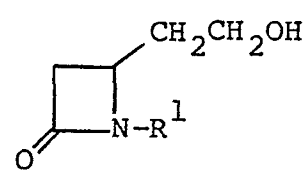

(I)

wherein $R^1$ is hydrogen atom or a protective group of amino group.

A bioactivity of the antibiotics derived from the compound (I) depends on the absolute configuration at C-4 position. The enantiomer of the compound (I) having a (4R) configuration is a preferable intermediate compound for producing thienamycin or its analogs. The antipode of the compound (I) having a (4S) configuration is also prepared by the process of the present invention by using the starting compound (II) having a (4R) configuration.

According to the process of the present invention, the protective groups of amino group include tri-organosilyl groups such as triisopropylsilyl,

t-butyldiphenylsilyl and t-butyldimethylsilyl, preferably t-butyldimethylsilyl. Alkyl or aralkyl groups having from 1 to 7 carbon atoms represented by $R^2$ in the compound (II) include straight or branched hydrocarbon groups such as methyl, ethyl, propyl, isopropyl, butyl, isobutyl, t-butyl, and benzyl, p-nitrobenzyl group, preferably methyl. The reducing agent is selected from metal hydrides complex such as lithium aluminum tetrahydride or lithium boron tetrahydride; a combination mixture of sodium or potassium boron tetrahydride with lithium halide such as lithium fluoride, chloride, bromide or iodide; a complex of borane with tetrahydrofuran, dimethyl sulfide, triethylamine, t-butylamine, N,N-dimethylaniline, or pyridine; preferably lithium boron tetrahydride.

The reduction is carried out, in general, at a temperature of from -70°C to 80°C. When lithium aluminum tetrahydride is used as a reducing agent, it is desirable to protect amide proton at N-1 position with a protective group as described above. The reduction is carried out, in general, with cooling at a temperature of from -80°C to 0°C, preferably from -30°C to 0°C in tetrahydrofuran or diethyl ether. The other reducing agent is used with or without the protective group on amide proton on N-1 position in the compound (II), and the reduction is carried out at a temperature of from 0°C to the reflux temperature of the used solvent. As a solvent is used tetrahydrofuran, 1,2-dimethoxyethane or diethylether. When a combination mixture of sodium or potassium boron tetrahydride with lithium halide is used as a reducing agent, 1,2-dimethoxyethane is preferably used as a solvent, and the reduction is carried out at the reflux temperature of the used solvent. The optimum molar ratio of the reducing agent to the compound (II) is from 1 to 10, preferably from 1 to 4.

It is unexpected and unobvious that such a highly reactive reducing agent as lithium alminum tetrahydride or lithium boron tetrahydride can be employed to convert the ester group in the compound (II) into the

alcohol group without cleaving the β-lactam ring.

According to the present invention, the optically active compound (I) is easily obtained by the reduction of the optically active ester (II) as mentioned above. The optically active compound (I) is useful compound for the preparation of thienamycin or its analogs. For example, the 2-hydroxyethyl side chain at C-4 position can be easily converted to 4-carboxymethyl group by oxidation, with or without previous introduction of 1-hydroxyethyl group to C-3 position to yield an optically active 4-carboxymethyl-2-azetidinones represented by the formula (III):

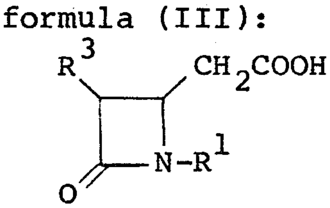

(III)

wherein $R^3$ is hydrogen atom, 1-hydroxyethyl, or 1-O-protected-oxyethyl group and $R^1$ is the same as that described above, from which thienamycin and various analogs are easily derived by a conventional method. As the oxidant is used Jones reagent, pyridinium chlorochromate or pyridinium dichromate.

A series of monocyclic β-lactam antibiotics such as monobactams having acylamido group at C-3 position and sulfo group ($SO_3H$) at N-1 position are also prepared from the compound (I) of the present invention by a conventional method.

According to a preferred process for preparing the compound (I), especially when $R^1$ is hydrogen atom and $R^2$ is methyl, the solution of the compound (II) in freshly distilled dry tetrahydrofuran is added to a suspended mixture of lithium boron tetrahydride and freshly distilled dry tetrahydrofuran dropwise at room temperature. After completion of the addition the resultant reaction mixture is stirred at room temperature for about an hour. The time course of the reduction is monitored by thin-layer chromatography (TLC) at intervals. When the starting compound cannot be detected on TLC, the

reaction mixture is cooled to $0^{o}C$ and the excess reducing agent is carefully destroyed by addition of ethyl acetate or a mixture of acetic acid and methanol. The desired product (I) is isolated by a usual manner described in the following examples.

The present invention is further illustrated by the following nonlimitative Examples.

## Example 1

Preparation of (4R)-(2-hydroxyethyl)-2-azetidinone (I)

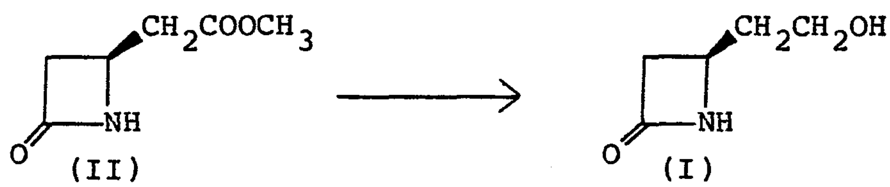

(II) (I)

To a suspended mixture of 0.75 g (34.4 mmoles) of $LiBH_4$ and 30 ml of freshly distilled dry tetrahydrofuran (hereinafter referred to as "THF") was added a solution containing 3.0 g (20.9 mmoles) of (4S)-methoxycarbonylmethyl-2-azetidinone (II) in 30 ml of freshly distilled dry THF dropwise at room temperature. The starting methyl ester (II) could not be detected on TLC in an hour's stirring at room temperature. A mixture of acetic acid and methanol (5 ml/3 ml) was carefully added to the cooled reaction mixture to destroy the excess of $LiBH_4$. The resultant reaction mixture was then filtered through Hyflo Super-Cel and the cake was washed sequentially with 20 ml of ethyl acetate and 40 ml of methanol. The filtrate and washings were combined and condensed to 7.93 g of yellow syrup which was chromatographed on a column of silica gel (Wakogel C-200: supplied from Wako Pure Chem. Ind. Ltd., 12 g, L/D = 210 mm/12 mmø, benzene-acetone (1:1, v/v)) to give 2.17 g of off-white crystals of the desired compound (I) (90 % of the theoretical amount).

mp: $64^{o}$ - $66^{o}C$

$[\alpha]_D^{25} = +12.7^{o}$ (c=2.0, MeOH)

TLC(Merck silica gel $F_{254}$): benzene-acetone (1:2, v/v)
Rf: compound (I) = 0.3; compound (II) = 0.6
(visualized with $I_2$ vapor)

Example 2

Preparation of (4R)-(2-hydroxyethyl)-2-azetidinone (I)

The procedure of Example 1 was repeated except that (4S)-benzyloxycarbonylmethyl-2-azetidinone was employed instead of (4S)-methoxycarbonylmethyl-2-azetidinone to give off-white crystals of the desired compound (I) (76 % of the theoretical amount).

Example 3

Preparation of (4S)-(2-hydroxyethyl)-2-azetidinone (I)

The procedure of Example 1 was repeated except that (4R)-methoxycarbonylmethyl-2-azetidinone was employed instead of (4S)-methoxycarbonylmethyl-2-azetidinone to give off-white crystals of the desired compound (I) (90 % of the theoretical amount).

$[\alpha]_D^{25} = -12.5^o$ (c=2.0, MeOH)

Example 4

Preparation of (4R)-(2-hydroxyethyl)-2-azetidinone (I)

The procedure of Example 1 was repeated except that a mixture of 2 mole eq. of $NaBH_4$ and 2 mole eq. LiCl was employed instead of $LiBH_4$, 1,2-dimethoxyethane was employed instead of THF and the reaction mixture was stirred for 3 hours at the reflux temperature to give off-white crystals of the desired compound (I) (74 % of the theoretical amount).

Example 5

Preparation of (4R)-(2-hydroxyethyl)-2-azetidinone (I)

The procedure of Example 1 was repeated except that a complex of borane with dimethyl sulfide (2M THF solution, 1.1 mole eq.) was employed instead of $LiBH_4$ and the reaction mixture was stirred for 2 hours at the reflux temperature to give off-white crystals of the desired

compound (I) (61 % of the theoretical amount).

Example 6

Preparation of (4R)-(2-hydroxyethyl)-1-t-butyldimethylsilyl-2-azetidinone (I)

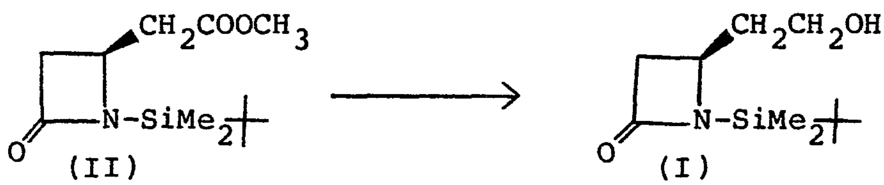
(II) (I)

To a mixture of 291 mg (7.66 mmoles) of $LiAlH_4$ and 30 ml of freshly distilled dry THF was added dropwise a solution containing 1.97 g (7.66 mmoles) of (4S)-methoxycarbonylmethyl-1-t-butyldimethyl-2-azetidinone (II) in 20 ml of freshly distilled dry THF in the course of ten minutes, while the reaction vessel was cooled in Dry Ice acetone bath and kept the inner temperature at $-40^{o}C$ to $-20^{o}C$. The reaction mixture was then stirred at $-30^{o}C$ for 30 minutes and the starting methyl ester (II) could not be detected on TLC. Then the reaction mixture was treated with the same manner as in Example 1 to give an yellow syrup which was chromatographed on silica gel column (Wakogel C-200, 25 g, benzene-acetone (95:5, v/v)) to give 1.60 g of colorless syrup of the desired compound (I) (91 % of the theoretical amount).

$[\alpha]_D^{25} = -52.9^{o}$ (c=2.0, $CHCl_3$)

TLC(Merck silica gel $F_{254}$): 10 % EtOH in $CH_2Cl_2$-n-Hexane (1:1, v/v)

Rf: compound (I) = 0.36; compound (II) = 0.6

WHAT WE CLAIM IS:

1. A process for the preparation of an optically active 4-(2-hydroxyethyl)-2-azetidinone represented by the formula (I):

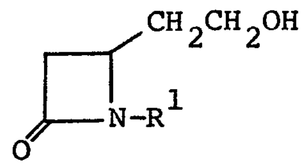

(I)

wherein $R^1$ is hydrogen atom or a protective group of amino group,
which comprises reacting an optically active carboxylic acid ester represented by the formula (II):

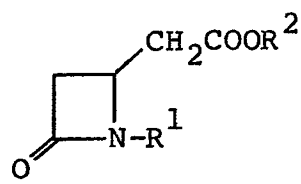

(II)

wherein $R^1$ is the same as that defined above; $R^2$ is alkyl or aralkyl group having from 1 to 7 carbon atoms, with a reducing agent.

2. The process of Claim 1 wherein the absolute configuration of the compound of the formula (I) is (4R).

3. The process of Claim 1 wherein the absolute configuration of the compound of the formula (I) is (4S).

4. The process of Claim 1 wherein $R^2$ is methyl group and $R^1$ is hydrogen atom.

5. The process of Claim 1 wherein $R^1$ is tri-organosilyl group.

6. The process of Claim 5 wherein the

tri-organosilyl group is triisopropylsilyl, t-butyldimethylsilyl or t-butyldiphenylsilyl.

7. The process of Claim 1 wherein the reducing agent is a metal hydride complex.

8. The process of Claim 7 wherein the metal hydride complex is lithium aluminum tetrahydride or lithium boron tetrahydride.

9. The process of Claim 7 wherein the metal hydride complex is a combination mixture of sodium boron tetrahydride with lithium halide or potassium boron tetrahydride with lithium halide.

10. The process of Claim 1 wherein the reducing agent is a complex of borane with tetrahydrofuran, dimethyl sulfide, triethylamine or pyridine.

11. The process of Claim 1 wherein the reduction of the compound of the formula (II) is carried out at a temperature of from -70$^{o}$C to 80$^{o}$C.

European Patent Office

# EUROPEAN SEARCH REPORT

0090340

Application number

EP 83 10 2866

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | EP-A-0 017 992 (MERCK) *Page 55, example 3* | 1 | C 07 D 205/08<br>C 07 F 7/10 |
| | --- | | |
| Y | EP-A-0 026 816 (MERCK) *Page 23* | 1,3,7 | |
| | ----- | | |
| | | | TECHNICAL FIELDS SEARCHED (Int. Cl. 3) |
| | | | C 07 D 205/00<br>C 07 F 7/00<br>C 07 D 487/00 |

The present search report has been drawn up for all claims

| Place of search | Date of completion of the search | Examiner |
|---|---|---|
| THE HAGUE | 14-06-1983 | CHOULY J. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons

& : member of the same patent family, corresponding document

EPO Form 1503. 03.82